# EUROPEAN PATENT APPLICATION

(11) **EP 1 106 149 A2**
(43) Date of publication of application: **13.06.2001**
(21) Application number: 00115324.6
(22) Date of filing: 14.07.2000
(51) Int. Cl.: A61F 13/00

(54) **Wound protective material**

(30) Priority: 10.12.1999 JP 35121099
(71) Applicant: Kurosu Ind. Co., Ltd., Amagasaki-shi, Hyogo (JP)
(72) Inventor: Okamoto, Nobuyuki, Amagasaki-shi, Hyogo (JP)
(74) Representative: Schieschke, Klaus, Dipl.-Ing.

(57) **Abstract**

A wound protective material which is stretchable, high in air permeability, adaptable to the size of wound, absorbs body fluids such as blood, is less likely to stick to a wound, and is hydrophobic or water-repellant. The wound protective material has a knitted fabric and a narrow adhesive layer provided at least at one end of the fabric and covered by a release sheet. The knitted fabric includes a hydrophobic or water-repellant substrate and a water-absorbent interwoven structure.

## Description

### BACKGROUND OF THE INVENTION

This invention relates to a protective tape to be wound around e.g. a finger to cover and protect a wound in the finger.

One typical way to protect or cure a wound in e.g. a finger is to cover the wound with gauze or absorbent cotton and wind a bandage over the gauze or cotton. To fasten the end of the bandage in position, a clip or an adhesive tape is needed. Although the bandage can also be fastened in position by splitting its end in half and knotting the split portions together, this method is uneconomical because a longer bandage is needed. The gauze or cotton tends to stick to the wound. Thus, when it is removed from the wound, part of the skin such as a scab tends to be removed together with the gauze. This gives the patient a lot of pain and also retards the healing of the wound.

An adhesive plaster in the shape of a tape or a strip of film with a pad stuck thereon also tends to stick rather strongly to the wound, so that it will give a pain to the patient when removed. Another problem of this type of plaster is its low air permeability. The wound thus tends to be oozy and turn white. Also, the adhesive may cause a rash. Such plasters are uneconomical too because plasters having pads of different sizes have to be prepared for wounds of different sizes.

Bandages, which have a good air permeability, are free of these problems inherent to adhesive plasters. And various improved bandages have been proposed. Japanese patent publication 5-329181 discloses a fibrous material for protecting wounds. But in this publication, no mention is made to how to control the moisture-absorbance and water-repellancy of this material. Japanese patent publication 6-6140 discloses a stretchable wound-protecting cloth having improved water repellancy. But no mention is made to its water-absorbance.

An object of this invention is to provide a wound protective tape which is stretchable, high in air permeability, can absorb body fluids such as blood, can retain moisture, and is hydrophobic and water-repellant.

### SUMMARY OF THE INVENTION

According to the invention, there is provided a wound protective material comprising a knitted fabric and an adhesive layer provided at at least one end of said fabric and covered by a release sheet, the knitted fabric comprising a hydrophobic or water-repellant substrate and a water-absorbent interwoven structure.

Other features and objects of the present invention will become apparent from the following description made with reference to the accompanying drawings, in which:

### BRIEF DESCRIPTION OF THE DRAWINGS

Fig. 1 is a plan view of a wound protective tape embodying the invention;

Fig. 2 is an enlarged view of the fabric of the tape of Fig. 1;

Fig. 3 is a similar view of a modified fabric; and

Fig. 4 is a cross-sectional view of the protective tape of Fig. 1 as wrapped around a finger.

### DETAILED DESCRIPTION OF THE PREFERRED EMBODIMENT

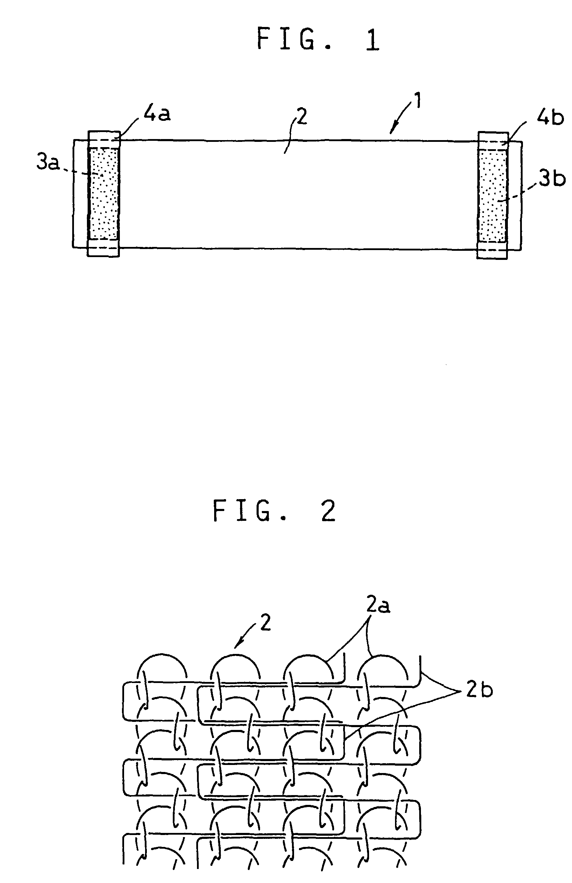

Embodiments are described with reference to the drawings. Fig. 1 shows a wound protective material 1 according to the invention, comprising a knitted fabric tape 2, and adhesive layers 3a, 3b provided at both ends of the fabric tape 2. The adhesive layers are covered by release paper sheets 4a, 4b. The adhesive layers 3a, 3b do not have to be wider than 5-20 mm. The release sheets preferably protrude from one or both ends of the adhesive layers so that they can be picked easily to remove them from the adhesive layers.

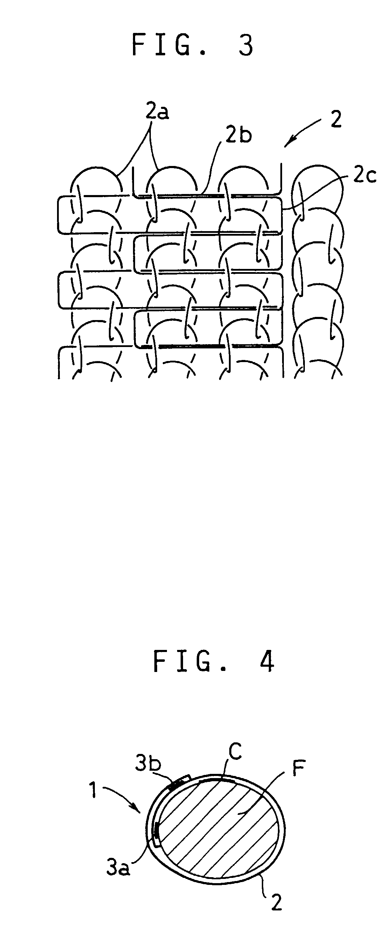

The fabric 2 comprises a substrate 2a made of a hydrophobic or water-repellant resilient yarn (such as SPANDEX: trade name) and an interwoven structure (pattern yarn) 2b. The interwoven structure may consist only of a water-absorbent natural or synthetic fiber yarn as shown in Fig. 2, or may further comprise a waterproof yarn 2c of a hydrophobic or water-repellant synthetic fiber to hold the water-absorbent fiber yarn in the substrate 2A as shown in Fig. 3. In either embodiment, the substrate has a warp-knitted structure.

Since the resilient yarn is used as warp, it hardly slips off the loop of the substrate, so that the edge can keep its resilience. Generally a knitted structure is liable to become loose at one end in the longitudinal direction, but not at the other end. The adhesive layer should be provided at such an end where the material is liable to become loose.

By inserting a required number of water-absorbent yarn, it is possible to control the water absorbance of the tape.

The adhesive layers 3a, 3b may be an adhesive applied to the fabric 2 or double-sided adhesive tapes stuck on the fabric. The adhesive used for the layers 3a, 3b is preferably a silicon or acrylic adhesive because such adhesive does not peel or melt in water during laundering and thus the tape is reusable after washing.

The protective tape 1 is made to be typically 1.5-20 cm wide and 5-100 cm long. A treating agent such as a disinfectant like acrinol or macrogol may be impregnated into or applied to part or the whole of the fabric 2 to such an extent as not to impair air permeability of the tape.

As shown in Fig. 4, the protective tape 1 is wrapped around e.g. a finger F to cover a wound C as shown in Fig. 4, and the adhesive layer 3b at the outer end of the fabric is stuck on the fabric by removing the release sheet 4b thereon. Before wrapping the tape around the finger, the release sheet 4a at the other end of the fabric may or may not be removed. Even if the release sheet 4a is removed as shown, the adhesive layer 3a will have no harmful effect on the skin of the finger and air permeability of the tape because the layer 3a as well as the layer 3b is narrow and small in area.

Since the protective tape according to the invention is formed of a fiber which is stretchable and high in air permeability, it will not wrinkle even if wrapped around an articulate portion of the body such as a finger. Also it is adaptable for as well as a small one, is easy to use, would not restrict the movement of e.g. a finger, and prevents oozing and whitening of the skin.

The adhesive layers are provided on the side of the fabric where the interwoven structure 2b is formed. Thus, when the tape is wrapped around an affected part, the hydrophobic or water-repellant substrate 2a is located outside the interwoven structure 2b and is kept out of direct contact with the wound, thus preventing entry of moisture from outside. On the other hand, the water-absorbent interwoven structure 2b, which is in direct contact with the wound, absorbs any oozing body fluid, thus preventing body fluid from congesting, hardening and sticking to the tape.

## Claims

1. A wound protective material comprising a knitted fabric in the form of a tape and an adhesive layer provided at at least one end of said fabric and covered by a release sheet, said knitted fabric comprising a hydrophobic or water-repellant substrate and a water-absorbent interwoven structure.

2. The wound protective material as claimed in claim 1 wherein said substrate is stretchable.

3. The wound protective material as claimed in claim 1 or 2 wherein said substrate has a warp-knitted structure and wherein said interwoven structure comprises a pattern yarn.

4. The wound protective material as claimed in any of claims 1-3 wherein a treating agent is impregnated into or applied to said knitted fabric.

5. The wound protective material as claimed in claim 1 wherein said adhesive layer comprises a silicon or acrylic adhesive.
